# EUROPÄISCHE PATENTANMELDUNG

(11) **EP 4 311 526 A1**
(43) Veröffentlichungstag der Anmeldung: **31.01.2024**
(21) Anmeldenummer: 23184113.1
(22) Anmeldetag: 07.07.2023
(51) Int. Cl.: A61F 2/46, A61B 17/15, A61F 2/38, A61F 2/30

(54) **FEMORALES PROBEKONDYLENIMPLANTAT**

(30) Priorität: 25.07.2022 DE 102022207580
(71) Anmelder: Aesculap AG, 78532 Tuttlingen (DE)
(72) Erfinder: FIRMBACH, Franz-Peter, 78576 Emmingen-Liptingen (DE); ANHORN, Svenja, 72535 Heroldstatt (DE)
(74) Vertreter: Patentanwälte Ruff, Wilhelm, Beier, Dauster & Partner mbB

(57) **Zusammenfassung**

Die Erfindung betrifft ein femorales Probekondylenimplantat (1, 1a, 1b, 1c, 1d) zur probeweisen Anbringung an einem resezierten Femur (F) bei einer Kniegelenkersatzoperation, aufweisend eine distale Vorderseite (V) mit einer medialen Kondylenfläche (2), welche zur Artikulation mit einem medialen Tibiaplateau (TPM) einer proximalen Tibia (T) eingerichtet ist, und mit einer lateralen Kondylenfläche (3), welche zur Artikulation mit einem lateralen Tibiaplateau (TPL) der proximalen Tibia eingerichtet ist, und aufweisend eine proximale Rückseite (R), wobei die Rückseite eben ist und eine flache Knochenkontaktfläche (4) bildet, welche zur Kontaktierung einer distalen Schnittfläche (SF) des distal resezierten Femurs eingerichtet ist.

## Beschreibung

Die Erfindung betrifft ein femorales Probekondylenimplantat zur probeweisen Anbringung an einem resezierten Femur bei einer Kniegelenkersatzoperation.

Bei einer Kniegelenkersatzoperation (englisch: Total Knee Arthroplasty (TKA)) werden abgenutzte oder anderweitig durch Krankheit oder einen Unfall beeinträchtigte Gelenkflächen des Femurs und/oder der Tibia durch künstliche Gelenkflächen einer Kniegelenkprothese ersetzt. Solche Kniegelenkprothesen umfassen üblicherweise eine Femurkomponente und eine Tibiakomponente. Die Femurkomponente wird am distalen Ende des Femurs implantiert. Die Tibiakomponente wird am proximalen Ende der Tibia implantiert.

Vor der Implantation der prothetischen Komponenten werden der distale Femur und die proximale Tibia reseziert. Hierfür bringt der Chirurg unterschiedliche Resektionsschnitte an und trennt Knochen- und/oder Knorpelmaterial von dem jeweiligen Knochen ab. Durch die Resektion wird der jeweilige Knochen in seiner Form an die aufzunehmende prothetische Komponente angepasst.

Die Resektion kann auf der Grundlage unterschiedlicher Konzepte ausgeführt werden. Ein Konzept zielt darauf ab, die Spannungen der Bänder des Knies bei der Gelenkbewegung ausgeglichen zu halten. Hierdurch soll eine bessere Funktion der Kniegelenkprothese gewährleistet werden. Dieses Konzept wird allgemein als "gap balancing" bezeichnet. Bei anderen Konzepten entfernt der Chirurg mittels der Resektion eine bestimmte Menge an Knochen- und/oder Knorpelmaterial. Solche Konzepte werden allgemein als "measured resection" bezeichnet. Die Ausrichtung der Resektionsschnitte in Bezug auf die Anatomie des Patienten bestimmt die spätere Ausrichtung der implantierten Komponenten und folglich auch die Orientierung der prothetischen Gelenkachsen. Die Ausrichtung der Resektionsschnitte ist daher von besonderer Bedeutung.

Bei der Ausrichtung der Resektionsschnitte werden in erster Linie drei Ansätze unterschieden: mechanisch, anatomisch und kinematisch. Bei der mechanischen Ausrichtung wird die proximale Tibia senkrecht zur Längsachse des Tibiaschafts reseziert. Die Resektion des distalen Femurs erfolgt entsprechend hieran angepasst. Erforderlichenfalls werden Bandentlastungen (englisch: ligament releases) durchgeführt. Bei der anatomischen Ausrichtung wird versucht, die Tibia in einem Varus-Winkel von 3° zu resezieren. Die Femurresektion und Bandentlastungen werden mit dem Ziel einer geraden Hüft-Knie-Knöchel-Achse des Beins durchgeführt. Ziel der kinematischen Ausrichtung (englisch: kinematic alignment, nachfolgend abgekürzt als KA) ist es, die künstlichen Gelenkflächen der prothetischen Komponenten auf dem Niveau der präarthritischen, defektfreien natürlichen Gelenkflächen zu implantieren.

Bei einer KA erfolgt die Ausrichtung der Resektionsschnitte oftmals ausgehend von dem distalen Femur. Die Resektion der proximalen Tibia erfolgt hieran angepasst. In diesem Zusammenhang wird auch von einer Übertragung der Ausrichtungen und/oder Schnitte gesprochen. Zu diesem Zweck sind spezielle chirurgische Instrumente bekannt, die auch als tibiales Ausricht- und/oder Übertragungswerkzeug (tibial cut alignment guide) bezeichnet werden. Solche Instrumente erlauben eine Übertragung der Ausrichtung der femoralen Resektionsschnitte auf die Tibia. Die Übertragung erfolgt üblicherweise nach einer wenigstens distalen Resektion des Femurs, bei welcher die distalen Kondylen abgetrennt werden. Die Übertragung kann in Extension oder Flexion erfolgen.

Vor diesem Hintergrund besteht ein grundsätzlicher Bedarf nach chirurgischen Instrumenten, Probeimplantaten und Implantaten zur Umsetzung des Kinematic Alignment. Im Speziellen besteht ein Bedarf nach Instrumenten und Probeimplantaten, die eine Übertragung der Ausrichtung der Resektionsschnitte von dem distalen Femur auf die proximale Tibia ermöglichen.

Aus der US 10,912,658 B2 ist ein femorales Probeimplantat bekannt und zur Verwendung bei einer Ausrichtung der tibialen Resektionsschnitte vorgesehen. Das bekannte Probeimplantat weist eine speziell gestaltete Rückseite auf, die zur Anbringung an einem vollständig resezierten Femur eingerichtet ist. Vollständig meint, dass die distalen, anterioren und posterioren Kondylen entfernt und zusätzliche Fasenschnitte angebracht sind. Eine der Rückseite des Probeimplantats gegenüberliegende Vorderseite weist Schnittstellen zur lösbaren Verbindung mit einem tibialen Übertragungswerkzeug auf.

Aufgabe der Erfindung ist es, eine verbesserte Übertragung der Ausrichtung der Resektionsschnitte im Rahmen des Kinematic Alignment zu ermöglichen.

Diese Aufgabe wird durch das Bereitstellen eines femoralen Probekondylenimplantats mit den Merkmalen des Anspruchs 1 gelöst.

Das erfindungsgemäße femorale Probekondylenimplantat ist zur probeweisen Anbringung an einem resezierten Femur bei einer Kniegelenkersatzoperation vorgesehen und weist auf: eine distale Vorderseite mit einer medialen Kondylenfläche, welche zur Artikulation mit einem medialen Tibiaplateau einer proximalen Tibia eingerichtet ist, und mit einer lateralen Kondylenfläche, welche zur Artikulation mit einem lateralen Tibiaplateau der proximalen Tibia eingerichtet ist. Zudem weist das erfindungsgemäßen femorale Probekondylenimplantat eine proximale Rückseite auf, wobei die Rückseite eben ist und eine flache Knochenkontaktfläche bildet, welche zur Kontaktierung einer distalen Schnittfläche des distal resezierten Femurs eingerichtet ist. Durch die erfindungsgemäße Lösung kann der proximale Tibiaschnitt vor dem Anbringen der anterioren und posterioren Femurschnitte ausgerichtet werden. Zu diesem Zweck ist die Rückseite des Probekondylenimplantats eben und bildet die besagte flache Knochenkontaktfläche. Die flache Knochenkontaktfläche ist zur Kontaktierung der distalen Schnittfläche des Femurs eingerichtet. Die distale Schnittfläche resultiert aus einem distalen Femurschnitt. Im Unterschied zu aus dem Stand der Technik bekannten Lösungen erlaubt die Erfindung eine verbesserte Ausrichtung der anterioren und posterioren Femurschnitte. Deren Ausrichtung kann erforderlichenfalls auch nach der Ausrichtung des Tibiaschnitts festgelegt und/oder korrigiert werden. Bei aus dem Stand der Technik bekannten Lösungen ist dies nicht ohne weiteres möglich, da die anterioren und posterioren Femurschnitte bereits vor dem Anbringen des Probeimplantats und der hierauf basierenden Ausrichtung des Tibiaschnitts angebracht sein müssen. Die beiden Kondylenflächen des Probekondylenimplantats ersetzen die abgetrennten natürlichen distalen Kondylen des Femurs. Die natürlichen anterioren und posterioren Femurkondylen werden dementgegen nicht ersetzt. Das Probekondylenimplantat weist keine Flächen auf, welche anstelle der natürlichen anterioren und posterioren Femurkondylen treten. In der Verwendung des Probekondylenimplantats sind die natürlichen anterioren und posterioren Femurkondylen üblicherweise nicht oder noch nicht abgetrennt. Die probeweise Anbringung erfolgt bei unterschiedlichen Ausgestaltungen auf unterschiedliche Weise, beispielsweise stoff-, kraft- und/oder formschlüssig.

Die in dieser Beschreibung verwendeten Lage- und Richtungsbezeichnungen beziehen sich auf den Körper eines Patienten, insbesondere dessen Femur, und sind insoweit gemäß ihrer üblichen anatomischen Bedeutung zu verstehen. Folglich bedeutet "anterior" vordere(r) oder vorne liegend, "posterior" hintere(r) oder hinten liegend, "medial" innere(r) oder innen liegend, "lateral" äußere(r) oder außen liegend, "proximal" zum Körperzentrum hin und "distal" vom Körperzentrum entfernt. Weiter bedeuten "proximodistal" entlang, vorzugsweise parallel zu, einer proximal-distal ausgerichteten Achse, "anteroposterior" entlang, vorzugsweise parallel zu, einer anterior-posterior ausgerichteten Achse und "mediolateral" entlang, vorzugsweise parallel zu, einer medial-lateral ausgerichteten Achse. Die besagten Achsen sind orthogonal zueinander ausgerichtet und können selbstverständlich in Bezug zu nicht mit der Anatomie des Patienten in Verbindung stehenden X-, Y- und Z-Achsen gesetzt werden. Beispielsweise kann die proximaldistale Achse alternativ als X-Achse bezeichnet werden. Die medial-laterale Achse kann als Y-Achse bezeichnet werden. Die anterior-posteriore Achse kann als Z-Achse bezeichnet werden. Zur verbesserten Veranschaulichung und der Einfachheit der Bezeichnungen wegen werden nachfolgend in erster Linie die besagten anatomischen Lage- und Richtungsbezeichnungen verwendet. Weiter werden Bezeichnungen wie "Vorderseite" in Bezug auf eine proximal gerichtete Blickrichtung verwendet. Dementgegen werden Bezeichnungen wie "Rückseite" in Bezug auf eine distal gerichtete Blickrichtung verwendet.

In Ausgestaltung der Erfindung ist wenigstens ein Befestigungsabschnitt vorhanden und zur lösbaren Befestigung an dem distalen Femur eingerichtet. Bei einer Ausgestaltung dient der wenigstens eine Befestigungsabschnitt einer Aufnahme eines gesonderten Befestigungselements, beispielsweise eines Nagels, einer Schraube oder dergleichen. Bei einer weiteren Ausgestaltung bildet der wenigstens eine Befestigungsabschnitt selbst ein solches Befestigungselement. Durch den wenigstens einen Befestigungsabschnitt kann insbesondere auf eine stoffschlüssige Anbringung des Probekondylenimplantats verzichtet werden. Dies kann die Verwendung des Probekondylenimplantats vereinfachen.

Bei einer Ausgestaltung der Erfindung ist der wenigstens eine Befestigungsabschnitt eine proximodistal zwischen der Vorderseite und der Rückseite längserstreckte Durchgangsbohrung, welche zur Aufnahme eines zylindrischen Befestigungsstifts eingerichtet ist. Der zylindrische Befestigungsstift kann ausgehend von der Vorderseite durch die Durchgangsbohrung in den distalen Femur eingebracht werden. Der zylindrische Befestigungsstift kann beispielsweise eine Schraube, ein Nagel oder dergleichen sein. Vorzugsweise sind wenigstens zwei Durchgangsbohrungen vorhanden. In diesem Fall ist vorzugsweise eine erste Durchgangsbohrung auf der medialen Kondylenfläche angeordnet und eine zweite Durchgangsbohrung ist auf der lateralen Kondylenfläche angeordnet.

In weiterer Ausgestaltung der Erfindung ist der wenigstens eine Befestigungsabschnitt ein proximal von der Rückseite aufragender zylindrischer Befestigungsstift. Bei dieser Ausgestaltung kann das Probekondylenimplantat zur Anbringung an dem resezierten Femur mit dem zylindrischen Befestigungsstift voran auf die distale Schnittfläche gedrückt werden. Hierbei dringt der zylindrische Befestigungsstift in den distal resezierten Femur ein und bewirkt eine kraftschlüssige lösbare Befestigung. Vorzugsweise ist der zylindrische Befestigungsstift an seinem proximalen Ende angespitzt. Vorzugsweise sind wenigstens zwei zylindrische Befestigungsstifte vorhanden. In diesem Fall ist ein erster zylindrischer Befestigungsstift rückseits der medialen Kondylenfläche angeordnet und ein zweiter zylindrischer Befestigungsstift ist rückseits der lateralen Kondylenfläche angeordnet. Bei dieser Ausgestaltung kann auf ein gesondertes Befestigungsmittel verzichtet werden. Das Befestigungsmittel, genauer der zylindrische Befestigungsstift, ist ein integraler Bestandteil und/oder einstückig angeformter Abschnitt des Probekondylenimplantats.

In weiterer Ausgestaltung der Erfindung weist die Vorderseite wenigstens einen ersten Kopplungsabschnitt auf, welcher eingerichtet ist zur kraft- und/oder formschlüssigen lösbaren Kopplung mit einem tibialen Ausrichtinstrument zur Ausrichtung eines Tibiaschnittblocks. In gekoppeltem Zustand ist das tibiale Ausrichtinstrument relativ zu dem Probekondylenimplantat vorzugsweise fest und/oder unbeweglich positioniert. Das Probekondylenimplantat, genauer: dessen Ausrichtung, dient als Referenz für die Ausrichtung des Tibiaschnittblocks und damit auch für die Ausrichtung des Tibiaschnitts. Die Ausrichtung wird mittels des angekoppelten tibialen Ausrichtinstruments von dem Probekondylenimplantat auf den Tibiaschnittblock übertragen. Bei unterschiedlichen Ausgestaltungen ist der wenigstens eine erste Kopplungsabschnitt unterschiedlich gestaltet, beispielsweise als Aufnahmebohrung, Aufnahmeaussparung, Aufnahmenut oder dergleichen. Die lösbare Kopplung kann eine Steck-, Rast-, Klemm- und/oder Schnappverbindung sein. Vorzugsweise sind wenigstens zwei erste Kopplungsabschnitte vorhanden und mediolateral voneinander beabstandet an der Vorderseite angeordnet.

In weiterer Ausgestaltung der Erfindung ist der wenigstens eine erste Kopplungsabschnitt eine in die Vorderseite eingebrachte und anteroposterior längserstreckte Aufnahmebohrung. Die anteroposteriore Längserstreckung erlaubt ein besonders einfaches Ankoppeln des tibialen Ausrichtinstruments. Bei einer Ausgestaltung ist die Aufnahmebohrung als Durchgangsbohrung ausgeführt. Bei einer weiteren Ausgestaltung ist die Aufnahmebohrung ein einends offenes Sackloch. In diesem Fall kann die Öffnung anterior oder posterior an der Vorderseite angeordnet sein. Vorzugsweise ist die Öffnung anterior angeordnet. Vorzugsweise sind wenigstens zwei Aufnahmebohrungen vorhanden und mediolateral voneinander beabstandet an der Vorderseite angeordnet.

In weiterer Ausgestaltung der Erfindung weist die Vorderseite wenigstens einen zweiten Kopplungsabschnitt auf, welcher eingerichtet ist zur kraft- und/oder formschlüssigen lösbaren Kopplung mit einem Kompensationselement zur maßlichen Kompensation eines Defekts des Tibiaplateaus. Dies ist eine besonders vorteilhafte Ausgestaltung der Erfindung. Defekte des Tibiaplateaus, d.h. arthritische oder anderweitig bedingte Knochen- und/oder Knorpelabnutzungen, können bei der Ausrichtung des Tibiaschnitts auf einfache Weise maßlich ausgeglichen werden. Zu diesem Zweck ist der wenigstens eine zweite Kopplungsabschnitt an der Vorderseite vorhanden und dient einer lösbaren Kopplung mit dem besagten Kompensationselement. In angekoppeltem Zustand überdeckt das Kompensationselement die mediale Kondylenfläche und/oder die laterale Kondylenfläche. Anstelle der jeweiligen Kondylenfläche gelangt das Kompensationselement zur Anlage an dem betreffenden Abschnitt des Tibiaplateaus. Bei unterschiedlichen Ausgestaltungen ist die lösbare Kopplung unterschiedlich ausgeführt und/oder der wenigstens eine zweite Kopplungsabschnitt unterschiedlich gestaltet. Die lösbare Kopplung kann beispielsweise eine Steck-, Klemm-, Rast-und/oder Schnappverbindung sein. Vorzugsweise weist die Vorderseite wenigstens zwei zweite Kopplungsabschnitte auf, welche mediolateral voneinander beabstandet angeordnet sind. In diesem Fall ist vorzugsweise einer der beiden zweiten Kopplungsabschnitte auf der medialen Kondylenfläche angeordnet und ein weiterer der beiden zweiten Kopplungsabschnitte ist auf der lateralen Kondylenfläche angeordnet.

Die Erfindung betrifft zudem ein Implantatsystem zur Verwendung bei einer Kniegelenkersatzoperation mit wenigstens einem femoralen Probekondylenimplantat gemäß der vorhergehenden Ausgestaltung und mit wenigstens einem Kompensationselement, welches zur kraft- und/oder formschlüssigen lösbaren Kopplung mit dem wenigstens einen zweiten Kopplungsabschnitt des Probekondylenimplantats und zur maßlichen Kompensation eines Defekts des Tibiaplateaus eingerichtet ist.

In weiterer Ausgestaltung der Erfindung sind mehrere unterschiedliche Kompensationselemente vorhanden, wobei die unterschiedlichen Kompensationselemente unterschiedliche proximodistale Dicken zur maßlichen Kompensation unterschiedlich stark ausgeprägter Defekte aufweisen. Die unterschiedlichen Kompensationselemente können wahlweise und je nach Ausprägung eines vorliegenden Defekts an der Vorderseite des Probekondylenimplantats angebracht werden.

Die Erfindung betrifft zudem ein Implantatsystem zur Verwendung bei einer Kniegelenkersatzoperation mit mehreren femoralen Probekondylenimplantaten nach einer der vorhergehenden Ausgestaltungen, wobei die mehreren Probekondylenimplantate unterschiedlich groß und zur probeweisen Anbringung an unterschiedlich großen Femurknochen eingerichtet sind. Mit anderen Worten weisen die mehreren Probekondylenimplantate unterschiedliche Größen auf. Die mehreren Probekondylenimplantate unterscheiden sich bei einer Ausgestaltung im Hinblick auf eine mediolaterale Abmessung der Vorderseite und/oder der Rückseite. Bei einer weiteren Ausgestaltung unterscheiden sich die mehreren Probekondylenimplantate alternativ oder zusätzlich hinsichtlich einer anteroposterioren Abmessung der Vorderseite und/oder Rückseite. Bei einer weiteren Ausgestaltung unterscheiden sich die mehreren Probekondylenimplantate alternativ oder zusätzlich im Hinblick auf eine zwischen der Vorderseite und der Rückseite erstreckte proximodistale Dicke.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematischer Darstellung und in proximaler Blickrichtung eine Ausführungsform eines erfindungsgemäßen femoralen Probekondylenimplantats in einem an einem distalen Femur angebrachten Zustand,
- Fig. 2, 3: unterschiedliche Ansichten einer exemplarischen intraoperativen Situation unter Verwendung des femoralen Probekondylenimplantats in einer posterioren Blickrichtung (Fig. 2) und einer medialen Blickrichtung (Fig. 3),
- Fig. 4: eine weitere Ausführungsform eines erfindungsgemäßen femoralen Probekondylenimplantats in einer schematischen, teilweise freigeschnittenen Seitenansicht in medialer Blickrichtung,
- Fig. 5: eine weitere Ausführungsform eines erfindungsgemäßen femoralen Probekondylenimplantats in einer schematischen Seitenansicht in medialer Blickrichtung,
- Fig. 6: eine weitere Ausführungsform eines erfindungsgemäßen femoralen Probekondylenimplantats in schematischer, teilweise freigeschnittener Seitenansicht in medialer Blickrichtung,
- Fig. 7: eine Ausführungsform eines erfindungsgemäßen Implantatsystems mit einer weiteren Ausführungsform eines erfindungsgemäßen femoralen Probekondylenimplantats und mit mehreren unterschiedlichen Kompensationselementen, wobei das Implantatsystem in einer schematischen, teilweise freigeschnittenen Seitenansicht in medialer Blickrichtung dargestellt ist, und
- Fig. 8: eine weitere Ausführungsform eines erfindungsgemäßen Implantatsystems mit mehreren unterschiedlichen femoralen Probekondylenimplantaten, wobei das Implantatsystem schematisch in einer proximalen Blickrichtung dargestellt ist.

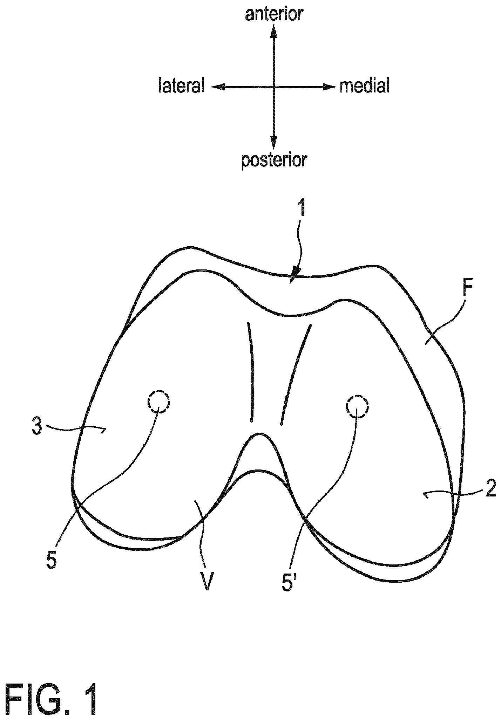

Gemäß den Fig. 1 bis 3 ist ein femorales Probekondylenimplantat 1 zur Verwendung bei einer Kniegelenkersatzoperation vorgesehen. Das femorale Probekondylenimplantat 1 wird nachfolgend der Kürze wegen als Kondylenimplantat bezeichnet.

Das Kondylenimplantat 1 ist zur probeweisen Anbringung an einem distalen Femur F eingerichtet und weist eine distale Vorderseite V und eine der Vorderseite V proximal gegenüberliegende Rückseite R auf.

In der Verwendung des Kondylenimplantats 1 ist die Rückseite R proximal orientiert und dem distalen Femur F zugewandt. Die Vorderseite V ist distal orientiert und einer proximalen Tibia, genauer: deren Tibiaplateau TP, zugewandt.

Die Vorderseite V weist eine mediale Kondylenfläche 2 und eine laterale Kondylenfläche 3 auf. Die mediale Kondylenfläche 2 ist zur Artikulation mit einem medialen Abschnitt des Tibiaplateaus TP eingerichtet. Dieser Abschnitt kann auch als mediales Tibiaplateau TPL bezeichnet werden. Die laterale Kondylenfläche 3 ist zur Artikulation mit einem lateralen Abschnitt des Tibiaplateaus TP eingerichtet. Dieser Abschnitt des Tibiaplateaus TP kann auch als laterales Tibiaplateau TPL bezeichnet werden.

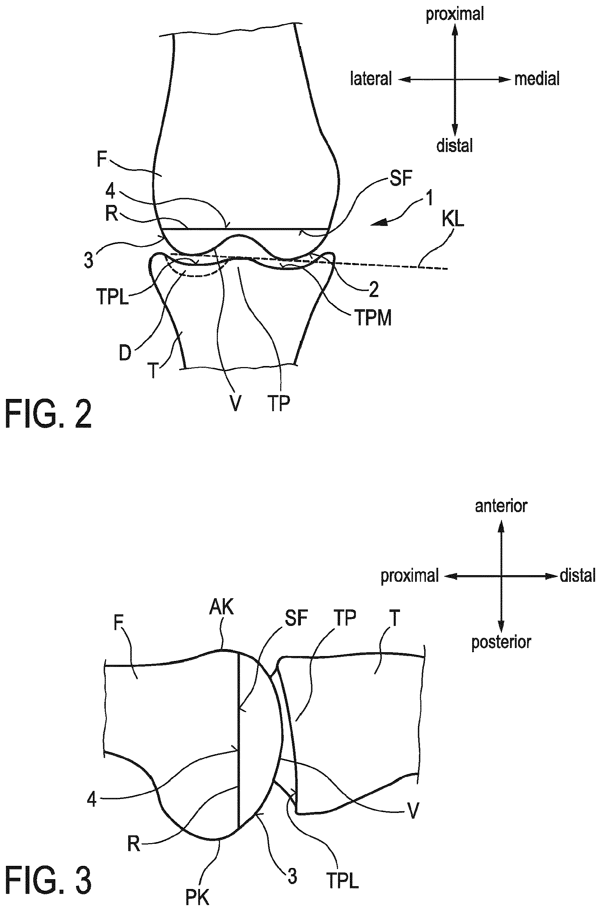

Die Rückseite R ist eben und bildet eine flache Knochenkontaktfläche 4. Die flache Knochenkontaktfläche 4 ist zur Kontaktierung einer distalen Schnittfläche SF des distal resezierten Femurs F eingerichtet. In der anhand der Fig. 1 bis 3 gezeigten intraoperativen Situation ersetzen die beiden Kondylenflächen 2, 3 des Kondylenimplantats 1 die natürlichen distalen Kondylenflächen des Femurs F. Diese wurden vor Anbringung des Kondylenimplantats 1 auf eine dem Fachmann bekannte Weise mittels eines distalen Femurschnitts abgetrennt. Der Femur F verfügt nach wie vor über seine anterioren Kondylenflächen AK und seine posterioren Kondylenflächen PK. Die anterioren Kondylenflächen AK und die posterioren Kondylenflächen PK werden - im Gegensatz zu den natürlichen distalen Kondylenflächen - mittels des Kondylenimplantats 1 nicht ersetzt. Die beiden Kondylenflächen 2, 3 simulieren eine distale Kondylenlinie KL des Femurs (siehe Fig. 2).

Die mediale Kondylenfläche 2 und die laterale Kondylenfläche 3 sind konvex gewölbt und in ihrer Form einer Form der natürlichen distalen Kondylen des Femurs F nachempfunden. Es versteht sich, dass die in den Figuren gezeigte Formgebung der Kondylenflächen 2, 3 als rein exemplarisch anzusehen ist.

Das Kondylenimplantat 1 wird lediglich probeweise an dem Femur F angebracht. Die Anbringung erfolgt bei unterschiedlichen Ausgestaltungen auf unterschiedliche Weise. Bei einer in den Figuren nicht gezeigten Ausführungsform ist eine stoffschlüssige Verbindung vorgesehen. Zu diesem Zweck kann die Rückseite R mit der distalen Schnittfläche SF stoffschlüssig zusammengefügt werden. Hierfür geeignete medizinische Klebstoffe oder dergleichen sind dem Fachmann bekannt.

Bei den in den Figuren gezeigten Ausführungsformen ist eine kraftschlüssige lösbare Befestigung vorgesehen. Zu diesem Zweck weist das Kondylenimplantat 1 wenigstens einen Befestigungsabschnitt 5 auf. Der Befestigungsabschnitt 5 ist bei unterschiedlichen Ausgestaltungen unterschiedlich ausgeführt.

Bei der gezeigten Ausführungsform weist das Kondylenimplantat 1 zwei Befestigungsabschnitte 5, 5' auf (siehe Fig. 1). Die beiden Befestigungsabschnitte 5, 5' sind mediolateral voneinander beabstandet angeordnet und können auch als lateraler Befestigungsabschnitt 5 und medialer Befestigungsabschnitt 5' bezeichnet werden. Die beiden Befestigungsabschnitte 5, 5' sind abgesehen von ihrer unterschiedlichen Anordnung identisch gestaltet. Weitere Details der Befestigungsabschnitte 5, 5' werden deshalb nachfolgend zur Vermeidung von Wiederholungen lediglich in Bezug auf einen der beiden Befestigungsabschnitte 5, 5' näher erläutert. Das diesbezüglich Gesagte gilt für beide Befestigungsabschnitte 5, 5`.

Das Kondylenimplantat 1 weist bei der gezeigten Ausführungsform einen einstückigen Aufbau auf. Alternativ denkbar ist eine mehrteilige Gestaltung. Als Werkstoff kommen für medizinische Anwendungen geeignete Metalle oder Kunststoffe infrage. Solche Werkstoffe sind dem Fachmann bekannt.

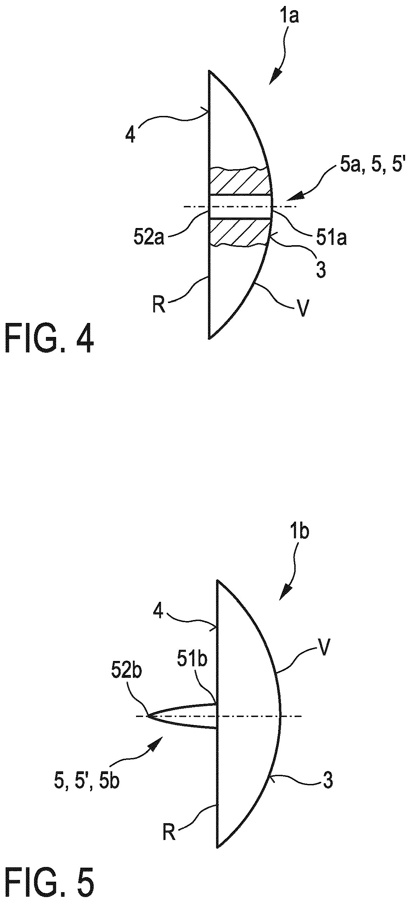

Die Fig. 4 bis 7 zeigen weitere Ausführungsformen erfindungsgemäßer femoraler Probekondylenimplantate 1a, 1b, 1c, 1d. Der Aufbau und die Funktionsweise der Kondylenimplantate 1a, 1b, 1c, 1d sind prinzipiell identisch mit dem Aufbau und der Funktionsweise des Kondylenimplantats 1 nach den Fig. 1 bis 3. Zur Vermeidung von Wiederholungen werden daher nachfolgend lediglich wesentliche Unterschiede der Kondylenimplantate 1a, 1b, 1c, 1d erläutert. Funktionsgleiche Abschnitte werden nicht bei jeder Ausführungsform beschrieben. Stattdessen wird auf das diesbezüglich im Zusammenhang mit dem Kondylenimplantat 1 nach den Fig. 1 bis 3 Gesagte verwiesen und ausdrücklich Bezug genommen.

Bei dem Kondylenimplantat 1a nach Fig. 4 ist der Befestigungsabschnitt 5 eine Durchgangsbohrung 5a. Die Durchgangsbohrung 5a ist zwischen der Vorderseite V und der Rückseite R durchgängig längserstreckt. Die Durchgangsbohrung 5a weist eine auf der Vorderseite V angeordnete distale Öffnung 51a und eine auf der Rückseite R angeordnete proximale Öffnung 52a auf. Die Durchgangsbohrung 5a ist entlang einer proximodistalen Achse durch das Kondylenimplantat 1a erstreckt. Die Durchgangsbohrung 5a ist zur Aufnahme eines in den Figuren nicht gezeigten zylindrischen Befestigungsstifts eingerichtet. Solche Befestigungsstifte sind dem Fachmann bekannt und können beispielsweise als Schraube, Nagel, Pin oder dergleichen gestaltet sein. Zur probeweisen Anbringung des Kondylenimplantats 1a an dem distalen Femur F wird die Rückseite R mit der distalen Schnittfläche SF kontaktiert. Hiernach wird der besagte zylindrische Befestigungsstift ausgehend von der Vorderseite V durch die distale Öffnung 51a in die Durchgangsbohrung 5a eingeführt und über die proximale Öffnung 52a in die distale Schnittfläche SF eingebracht.

Bei dem Kondylenimplantat 1b nach Fig. 5 ist der Befestigungsabschnitt 5 ein zylindrischer Befestigungsstift 5b. Der Befestigungsstift 5b ragt in proximaler Richtung von der Rückseite R ab. Der Befestigungsstift 5b weist ein distales Ende 51b und ein proximales Ende 52b auf. Der Befestigungsstift 5b ist zwischen dem distalen Ende 51b und dem proximalen Ende 52b längserstreckt. Das distale Ende 51b ist fest mit der Rückseite R verbunden. Das proximale Ende 52b ist vorliegend angespitzt. Bei der gezeigten Ausführungsform ist der Befestigungsstift 5b einstückig zusammenhängend mit den übrigen Abschnitten des Kondylenimplantats 1b. Bei einer in den Figuren nicht gezeigten Ausführungsform ist der Befestigungsstift ein gesondert gefertigtes und hiernach fest mit der Rückseite zusammengefügtes Bauteil. Zur probeweisen Anbringung des Kondylenimplantats 1 wird die Rückseite R gegenüber der distalen Schnittfläche F positioniert und der Befestigungsstift 5b wird manuell in die Knochenstruktur des Femurs F eingedrückt.

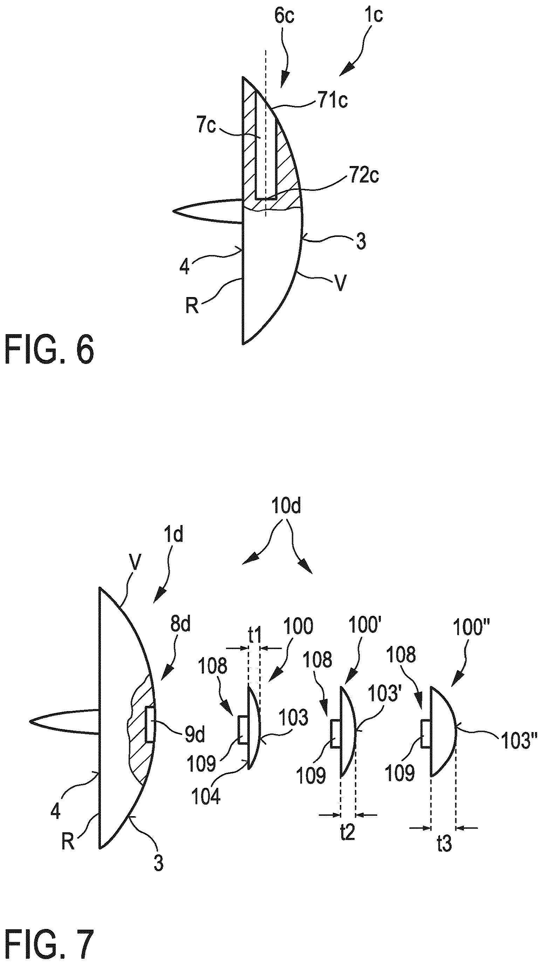

Bei dem Kondylenimplantat 1c nach Fig. 6 ist ein erster Kopplungsabschnitt 6c vorhanden. Der erste Kopplungsabschnitt 6c dient einer kraft- und/oder formschlüssigen lösbaren Kopplung mit einem tibialen Ausrichtinstrument zur Ausrichtung eines Tibiaschnittblocks. Solche tibialen Ausrichtinstrumente sind dem Fachmann bekannt und dienen einer Übertragung der Orientierung der distalen Schnittfläche SF auf die proximale Tibia T. Der erste Kopplungsabschnitt 6c ist an der Vorderseite V angeordnet. Bei der gezeigten Ausführungsform ist der erste Kopplungsabschnitt 6c eine Aufnahmebohrung 7c. Die Aufnahmebohrung 7c ist anteroposterior längserstreckt und weist vorliegend eine anteriore Öffnung 71c und einen posterioren Anschlag 72c auf. Die Aufnahmebohrung 7c ist zwischen der Öffnung 71c und dem Anschlag 72c längserstreckt und insoweit als Sackbohrung ausgeführt. Die Aufnahmebohrung 7c ist zur Aufnahme eines komplementären Kopplungszapfens des besagten tibialen Ausrichtinstruments eingerichtet. Das Kondylenimplantat 1c weist vorliegend einen weiteren, in Fig. 6 nicht ersichtlichen, ersten Kopplungsabschnitt auf. Dieser weitere Kopplungsabschnitt ist medial von dem in Fig. 6 gezeigten ersten Kopplungsabschnitt 6c beabstandet und im Bereich der medialen Kondylenfläche 2 angeordnet.

Das Kondylenimplantat 1d nach Fig. 7 weist wenigstens einen zweiten Kopplungsabschnitt 8d auf. Der zweite Kopplungsabschnitt 8d ist zur kraft- und/oder formschlüssigen lösbaren Kopplung mit einem Kompensationselement 100 eingerichtet. Das Kompensationselement 100 dient zur maßlichen Kompensation eines Defekts D des Tibiaplateaus TP (siehe Fig. 2). Zusammen mit dem wenigstens einen Kompensationselement 100 bildet das Kondylenimplantat 1d ein Implantatsystem 10d.

Der zweite Kopplungsabschnitt 8d ist bei der gezeigten Ausführungsform im Bereich der lateralen Kondylenfläche 3 angeordnet. Das Kondylenimplantat 1d weist vorliegend einen weiteren zweiten Kopplungsabschnitt auf, welcher in Fig. 7 nicht ersichtlich ist. Dieser weitere zweite Kopplungsabschnitt ist im Bereich der medialen Kondylenfläche 2 angeordnet.

Zur lösbaren Kopplung mit dem Kondylenimplantat 1d weist das Kompensationselement 100 einen komplementären Kopplungsabschnitt 108 auf. Der komplementäre Kopplungsabschnitt 108 ist an einer Rückseite des Kompensationselements 100 angeordnet.

Bei der gezeigten Ausführungsform ist der zweite Kopplungsabschnitt 8d eine Rastaufnahme 9d. Dementsprechend ist der komplementäre Kopplungsabschnitt 108 des Kompensationselements 100 ein komplementärer Rastabschnitt 109. Die Rastaufnahme 9d ist in proximaler Richtung in die Vorderseite V eingesenkt. Der komplementäre Rastabschnitt 109 ragt proximal von der Rückseite des Kompensationselements 100 ab.

Das Kompensationselement 100 weist eine distale Kontaktfläche 103 und eine proximale Rückfläche 104 auf. Die Kontaktfläche 103 ist an einer Vorderseite des Kompensationselements angeordnet. Die Kontaktfläche 103 ist zur Artikulation mit dem Tibiaplateau TP eingerichtet. Die Kontaktfläche 103 ist konvex. Die Kontaktfläche 103 ist kleiner als die Kondylenflächen 2, 3. Je nachdem, ob das Kompensationselement 100 lateral oder medial an der Vorderseite V des Kondylenimplantats 1 angebracht ist, kontaktiert die Kontaktfläche 103 das mediale Tibiaplateau TPM oder das laterale Tibiaplateau TPL. In angebrachtem Zustand liegt die Rückfläche 104 an der Vorderseite V des Kondylenimplantats 1d an.

Bei der gezeigten Ausführungsform weist das Implantatsystem 10d mehrere unterschiedliche Kompensationselemente 100, 100', 100" auf. Diese werden auch als erstes Kompensationselement 100, zweites Kompensationselement 100' und drittes Kompensationselement 100" bezeichnet.

Die mehreren Kompensationselemente 100, 100', 100" sind gegeneinander austauschbar mit dem Kondylenimplantat 1d verbindbar. Dementsprechend weisen das zweite und dritte Kompensationselement 100', 100" jeweils einen Kopplungsabschnitt/Rastabschnitt 108, 109 auf, welcher identisch zu dem Kopplungsabschnitt 108/Rastabschnitt 109 des ersten Kompensationselements 100 ist.

Die Kompensationselemente 100, 100`, 100" unterscheiden sich hinsichtlich einer proximodistalen Dicke t1, t2, t3 und dienen zur maßlichen Kompensation unterschiedlich stark ausgeprägter Defekte. Je nach Ausprägung des Defekts kommt ein vergleichsweise dünnes oder ein vergleichsweise dickes Kompensationselement zum Einsatz.

Im Speziellen weist das erste Kompensationselement 100 eine erste Dicke t1 auf. Das zweite Kompensationselement 100' weist eine zweite Dicke t2 auf. Das dritte Kompensationselement 100 weist eine dritte Dicke t3 auf. Vorliegend ist die erste Dicke t1 kleiner als die zweite Dicke t2 und die dritte Dicke t3. Die zweite Dicke t2 ist größer als die erste Dicke t1 und kleiner als die dritte Dicke t3. Die dritte Dicke t3 ist größer als die erste Dicke t1 und als die zweite Dicke t2.

Bei der gezeigten Ausführungsform ist ein nicht näher bezeichneter Außendurchmesser der Kompensationselemente 100, 100', 100" identisch. Aufgrund der unterschiedlichen Dicken t1, t2, t3 sind die jeweiligen Kontaktflächen 103, 103', 103" unterschiedlich stark gewölbt. Alternativ sind Kompensationselemente mit unterschiedlichen Außendurchmessern denkbar.

Es versteht sich, dass die gezeigte Anzahl an Kompensationselementen rein exemplarisch ist. Selbstverständlich kann das Implantatsystem 10d mehr oder weniger als die gezeigten drei Kompensationselemente 100, 100', 100" aufweisen.

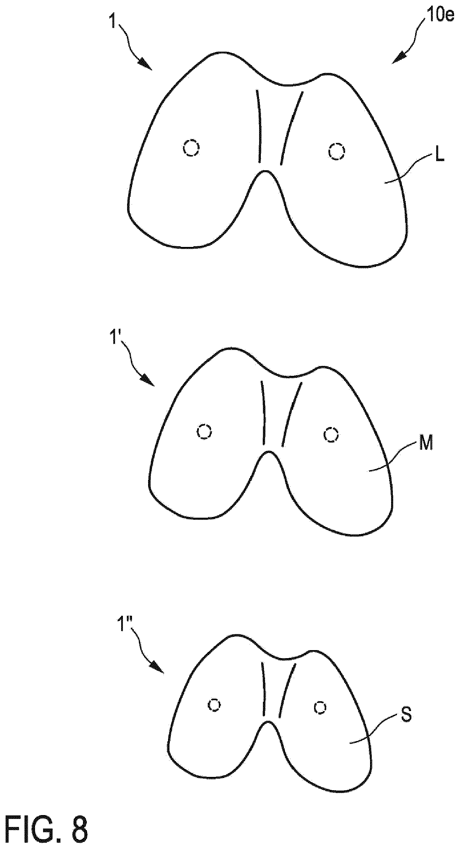

Fig. 8 zeigt eine weitere Ausführungsform eines erfindungsgemäßen Implantatsystems 10e. Das Implantatsystem 10e umfasst mehrere unterschiedliche Probekondylenimplantate 1, 1', 1", die nachfolgend auch als erstes Kondylenimplantat 1, zweites Kondylenimplantat 1' und drittes Kondylenimplantat 1" bezeichnet werden. Die unterschiedlichen Kondylenimplantate 1, 1', 1" weisen unterschiedliche Größen L, M, S auf. Die unterschiedlich großen Kondylenimplantate 1, 1', 1" sind zur probeweisen Anbringung an unterschiedlich großen Femurknochen vorgesehen.

Bei der gezeigten Ausführungsform unterscheiden die Kondylenimplantate 1, 1', 1" sich hinsichtlich ihrer mediolateralen und/oder anteroposterioren Abmessungen. Zudem sind die proximodistalen Abmessungen der Kondylenimplantate 1, 1', 1" unterschiedlich, was jedoch in Fig. 8 nicht ersichtlich ist. Die Größen L, M, S können auch als large, medium und small interpretiert werden.

Die Merkmale der Kondylenimplantate 1, 1a, 1b, 1c sowie 1d sind beliebig zu weiteren Merkmalskombinationen miteinander kombinierbar. Entsprechendes gilt sinngemäß für die Merkmale der Implantatsysteme 10d, 10e. Beispielsweise weist ein in den Figuren nicht gezeigtes erfindungsgemäßes Kondylenimplantat wenigstens einen ersten Kopplungsabschnitt und einen zweiten Kopplungsabschnitt auf. Zudem versteht sich, dass die Befestigungsabschnitte der Kondylenimplantate 1c, 1d, anders als in den Figuren gezeigt, jeweils als Durchgangsbohrung gestaltet sein können. Zudem weist ein in den Figuren nicht gezeigtes erfindungsgemäßes Implantatsystem zum einen unterschiedlich große Kondylenimplantate und zum anderen mehrere unterschiedliche Kompensationselemente auf, die wahlweise an jedem der unterschiedlichen Kondylenimplantate anbringbar sind.

## Patentansprüche

1. Femorales Probekondylenimplantat (1, 1a, 1b, 1c, 1d) zur probeweisen Anbringung an einem resezierten Femur (F) bei einer Kniegelenkersatzoperation, aufweisend
eine distale Vorderseite (V) mit einer medialen Kondylenfläche (2), welche zur Artikulation mit einem medialen Tibiaplateau (TPM) einer proximalen Tibia (T) eingerichtet ist, und mit einer lateralen Kondylenfläche (3), welche zur Artikulation mit einem lateralen Tibiaplateau (TPL) der proximalen Tibia (T) eingerichtet ist,
und aufweisend eine proximale Rückseite (R),
wobei die Rückseite (R) eben ist und eine flache Knochenkontaktfläche (4) bildet, welche zur Kontaktierung einer distalen Schnittfläche (SF) des distal resezierten Femurs (F) eingerichtet ist.

2. Femorales Probekondylenimplantat (1, 1a, 1b, 1c, 1d) nach Anspruch 1, **dadurch gekennzeichnet, dass** wenigstens ein Befestigungsabschnitt (5, 5') vorhanden und zur lösbaren Befestigung an dem distalen Femur (F) eingerichtet ist.

3. Femorales Probekondylenimplantat (1a) nach Anspruch 2, **dadurch gekennzeichnet, dass** der wenigstens eine Befestigungsabschnitt (5, 5') eine proximodistal zwischen der Vorderseite (V) und der Rückseite (R) längserstreckte Durchgangsbohrung (5a) ist, welche zur Aufnahme eines zylindrischen Befestigungsstifts eingerichtet ist.

4. Femorales Probekondylenimplantat (1b) nach Anspruch 2, **dadurch gekennzeichnet, dass** der wenigstens eine Befestigungsabschnitt (5, 5') ein proximal von der Rückseite (R) aufragender zylindrischer Befestigungsstift (5b) ist.

5. Femorales Probekondylenimplantat (1, 1a, 1b, 1c, 1d) nach einem der vorhergehenden Ansprüche, **dadurch gekennzeichnet, dass** die Vorderseite (V) wenigstens einen ersten Kopplungsabschnitt (6c) aufweist, welcher eingerichtet ist zur kraft- und/oder formschlüssigen lösbaren Kopplung mit einem tibialen Ausrichtinstrument zur Ausrichtung eines Tibiaschnittblocks.

6. Femorales Probekondylenimplantat (1, 1a, 1b, 1c, 1d) nach Anspruch 5, **dadurch gekennzeichnet, dass** der wenigstens eine erste Kopplungsabschnitt (6c) eine in die Vorderseite (V) eingebrachte und anteroposterior längserstreckte Aufnahmebohrung (7c) ist.

7. Femorales Probekondylenimplantat (1, 1a, 1b, 1c, 1d) nach einem der vorhergehenden Ansprüche, **dadurch gekennzeichnet, dass** die Vorderseite (V) wenigstens einen zweiten Kopplungsabschnitt (8d) aufweist, welcher eingerichtet ist zur kraft- und/oder formschlüssigen lösbaren Kopplung mit einem Kompensationselement (100) zur maßlichen Kompensation eines Defekts (D) des Tibiaplateaus (TP).

8. Implantatsystem (10d) mit wenigstens einem femoralen Probekondylenimplantat (1, 1a, 1b, 1c, 1d) nach Anspruch 7 und mit wenigstens einem Kompensationselement (100), welches zur kraft- und/oder formschlüssigen lösbaren Kopplung mit dem wenigstens einen zweiten Kopplungsabschnitt (8d) des Probekondylenimplantats (1, 1a, 1b, 1c, 1d) und zur maßlichen Kompensation eines Defekts (D) des Tibiaplateaus (TP) eingerichtet ist.

9. Implantatsystem (10d) nach Anspruch 8, **dadurch gekennzeichnet, dass** mehrere unterschiedliche Kompensationselemente (100, 100', 100") vorhanden sind, wobei die unterschiedlichen Kompensationselemente (100, 100`, 100") unterschiedliche proximodistale Dicken (t1, t2, t3) zur maßlichen Kompensation unterschiedlich stark ausgeprägter Defekte aufweisen.

10. Implantatsystem (10e) mit mehreren femoralen Probekondylenimplantaten (1, 1a, 1b, 1c, 1d) nach einem der Ansprüche 1 bis 8, wobei die mehreren Probekondylenimplantate (1, 1a, 1b, 1c, 1d) unterschiedlich groß und zur probeweisen Anbringung an unterschiedlich großen Femurknochen eingerichtet sind.
